(19) Europäisches Patentamt
European Patent Office
Office européen des brevets

(11) **EP 1 493 385 B1**

(12) **FASCICULE DE BREVET EUROPEEN**

(45) Date de publication et mention
de la délivrance du brevet:
**06.06.2007 Bulletin 2007/23**

(51) Int Cl.:
*A61B 5/11* *(2006.01)* *A61B 5/0205* *(2006.01)*

(21) Numéro de dépôt: **04103080.0**

(22) Date de dépôt: **30.06.2004**

(54) **Détecteur portatif pour mesurer des mouvements d'une personne**

Tragbarer Detektor zur Messung von Personenbewegungen

Portable detector device for measuring movements of a person

(84) Etats contractants désignés:
**AT BE BG CH CY CZ DE DK EE ES FI FR GB GR
HU IE IT LI LU MC NL PL PT RO SE SI SK TR**

(30) Priorité: **02.07.2003 FR 0350285**

(43) Date de publication de la demande:
**05.01.2005 Bulletin 2005/01**

(73) Titulaire: **COMMISSARIAT A L'ENERGIE
ATOMIQUE
75015 Paris (FR)**

(72) Inventeurs:
• **Guillemaud, Régis
38700, La Tronche (FR)**
• **Caritu, Yanis
38100, Grenoble (FR)**

(74) Mandataire: **Poulin, Gérard et al
Société BREVATOME
3, rue du Docteur Lancereaux
75008 Paris (FR)**

(56) Documents cités:
WO-A-02/061704 WO-A-03/005893
US-A- 5 935 081 US-B1- 6 477 421
US-B1- 6 491 647

EP 1 493 385 B1

## Description

Le sujet de cette invention est un détecteur portatif destiné à mesurer des mouvements de la personne porteuse.

Un art antérieur abondant existe pour mesurer des signaux cardiaques ou autres au moyen d'un détecteur qu'on colle sur le patient. Des capteurs de mouvement comme des accéléromètres ont ainsi été proposés pour suivre le mouvements de la cage thoracique et en déduire le rythme cardiaque. De tels détecteurs ont cependant été réservés à des conditions particulières d'état ou de posture du patient ; en général, une absence d'effort ou de mouvement est nécessaire pour donner une mesure fiable, qui ne soit pas brouillée par des composantes d'autre origine du signal de mouvement, qui pourraient être prépondérantes à cause de la petitesse des mouvements d'origine cardiaque.

On a aussi appliqué des capteurs de mouvement à divers endroits du corps pour suivre les personnes porteuses et parfois déterminer un état de sommeil, une chute, etc. La complexité des postures et des niveaux d'activité humaine ne se prête pas à une analyse réelle de l'activité avec les détecteurs usuels, qui sont plutôt réservés à la détection d'un seul genre d'événements et programmés pour ignorer les autres, dans la mesure du possible.

Il serait intéressant, par exemple, de compléter un détecteur de chute par un détecteur de mesures physiologiques pour vérifier l'état du patient après la chute, mais cela n'est possible qu'avec des détecteurs respectifs tous deux portés par le patient ce qui est inconfortable.

Un détecteur portatif perfectionné est proposé ici et comprend d'abord des moyens de traitement du signal permettant de distinguer une composante du signal, due à une activité extérieure du sujet porteur, d'au moins une composante du signal due à une activité physiologique (les battements du coeur ou le souffle notamment). Un tel dispositif est toutefois décrit par US-A-5 935 081.

Des objets de l'invention sont :

- de fournir un détecteur distinguant des composantes de signaux de niveaux bien différents et susceptible de varier fortement avec le temps ;
- de le faire à partir de mesures faites par des mêmes capteurs de mouvement ;
- de fournir un tel détecteur qui soit de structure unitaire et de petite taille ;
- d'offrir une capacité accrue de mesure et de diagnostic d'états physiologiques, en limitant les durées où les mesures ne doivent pas être considérées ;
- d'offrir une détermination plus universelle d'états de posture et d'activité du porteur en en discernant un plus grand nombre.

Il convient d'éviter qu'une détection incorrecte ne conduise à une alerte inutile. Une telle situation peut advenir avec certains mouvements extérieurs particulièrement brusques qui en pratique empêchent une détection convenable des mouvements d'origine physiologique. Il est donc utile et caractéristique de l'invention d'ajouter au détecteur un module reconnaissant une telle situation d'après des critères dépendant de la composante d'activité extérieure, et qui décide alors une invalidation temporaire de l'estimation de la composante due à l'activité physiologique.

Les battements du coeur et le souffle sont des mouvements périodiques, dont l'intensité et la fréquence varient d'après le niveau d'activité du porteur dans des conditions particulières. Les mouvements dus à l'activité extérieure du porteur sont généralement à basse fréquence ; mais n'étant pas périodiques, ils sont compris dans une bande de fréquence plus large, et leur intensité peut varier fortement. En raison de ces variations de fréquence et plus encore des recouvrements de bandes de fréquence associées à ces différents mouvements, il est impossible de les séparer par de simples filtrages du signal. On a cependant obtenu des résultats satisfaisants en appliquant un filtre non-stationnaire au signal ou aux signaux de mouvement et en soustrayant le signal filtré du signal d'origine (le signal brut ou bien un signal ayant subi un filtrage préliminaire pour éliminer le bruit) : les signaux d'origine physiologique ressortent alors assez bien.

Un autre écueil provient de la sensibilité des mesures à la posture corporelle prise par le porteur, puisque l'accélération de la gravité, qui intervient dans les mesures accélérométriques et doit y être corrigée, est perçue avec une intensité qui dépend de cette posture, et que les mesures de l'activité physiologique donnent des valeurs d'accélération beaucoup plus faible. On préconise d'ajouter des indicateurs de la position du porteur, notamment des magnétomètres mesurant la direction du champ magnétique ambiant, afin de bien déterminer la posture de la personne porteuse et de choisir certains seulement des signaux de mouvement, tout en écartant ceux qui sont trop affectés par la gravité, pour le traitement conforme à l'invention. Ce perfectionnement est utile avant tout quand plusieurs capteurs mesurent autant de mouvements du porteur dans des directions différentes. Une situation courante consiste en l'emploi de trois capteurs mesurant des mouvements dans des directions perpendiculaires, usuellement d'avance, de côté et d'élévation du porteur.

Une réalisation de l'invention sera maintenant décrite plus complètement en liaison aux figures. La figure 1 illustre la place du détecteur sur la personne porteuse, la figure 2 le détecteur dans son ensemble et la figure 3 le système de traitement.

EP 1 493 385 B1

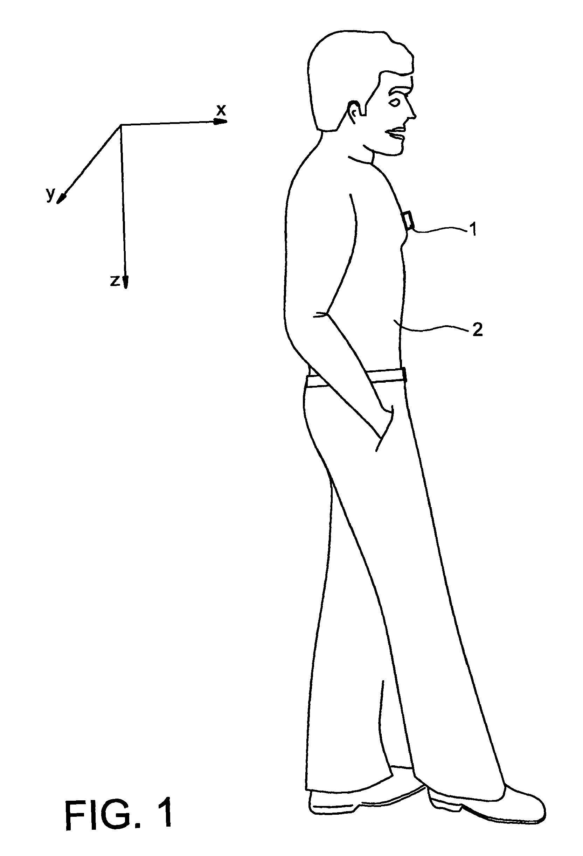

La figure 1 montre que le détecteur, portant la référence 1, est placé sur la poitrine d'un porteur 2. Il pourrait être placé sur l'abdomen ou ailleurs. Le détecteur 1 est miniature, ce qui lui permet d'être porté avec confort et sans presque être aperçu, contrairement à d'autres. Les axes X, Y et Z sont introduits pour la commodité de l'explication et définissent un repère lié au porteur 2, l'axe X étant dirigé vers l'avant, l'axe Z vers les pieds et l'axe Y vers la droite.

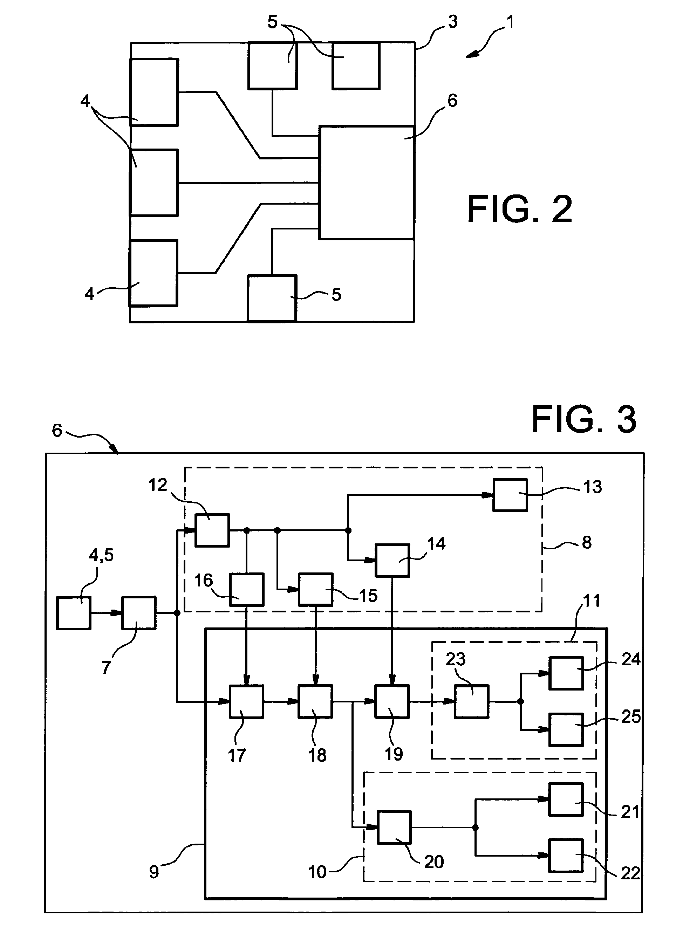

D'après la figure 2, le détecteur 1 peut comporter un boîtier 3 contenant trois accéléromètres portant tous la référence 4, trois magnétomètres portant tous la référence 5 et un système de traitement 6 auquel accéléromètres 4 et magnétomètres 5 sont reliés par des fils de manière à lui fournir leurs signaux. Les accéléromètres 4 mesurent chacun une composante d'accélération du mouvement de la poitrine du porteur 2 dans un des axes X, Y et Z respectivement en fonction de la direction de la gravité ; les magnétomètres 5 font de même en fonction de la direction du champ magnétique terrestre. Le détecteur 1 est maintenu à une orientation constante contre la peau ou un habit du porteur 2 par de la colle, une couture, une bande de serrage, ou tout autre moyen convenable. La description porte maintenant sur l'unité de traitement 6 à l'aide de la figure 3.

Les signaux issus des accéléromètres 4 ou des magnétomètres 5 passent chacun par un module de normalisation 7 et sont transmis à deux modules de calcul 8 et 9 travaillant à la fois en parallèle et en interaction dont le premier (8) calcule la composante des signaux qui est due à l'activité extérieure du porteur 2 et le second (9) calcule la composante des signaux due à l'activité physiologique ; ce second module 9 comprend un sous-module 10 affecté aux mouvements dus au battement du coeur et un sous-module 11 affecté aux mouvements dus au souffle respiratoire.

Le premier module de calcul 8 comprend un filtre passe-bas 12 qui transmet le signal issu du module de normalisation 7 à un dispositif d'analyse d'activité 13, à un dispositif d'analyse de posture 14, à un dispositif d'analyse de niveau d'activité 15 et un dispositif d'estimation 16 de la composante d'activité. Le signal issu du module de normalisation 7 parvient aux sous-modules 10 et 11 après être passé par un soustracteur 17, un module de validation 18, et encore, pour le sous-module 11, par un dispositif de choix 19. Le sous-module 10 comprend un dispositif d'extraction de la composante cardiaque 20, un dispositif de calcul de fréquence 21 et un dispositif d'examen 22. Le sous-module 11 comprend un dispositif d'extraction de la composante de respiration 23, un dispositif de calcul de fréquence 24 et un dispositif de sortie 25.

Ces différents éléments seront décrits successivement en détail. Le dispositif de normalisation 7 est d'un genre ordinaire qui permet de calibrer les signaux, par exemple selon une loi linéaire, pour fournir en sortie des signaux normalisés, qui sont proportionnels à l'accélération qu'ils subissent. Le filtre passe-bas 12 sert à éliminer les fréquences élevées du signal, qui n'expriment en pratique que des bruits. Le dispositif d'analyse d'activité 13 n'est pas indispensable et son contenu peut dépendre des genres d'activités qu'on cherche à diagnostiquer, comme la chute, le sommeil, la marche, le changement de position ou autres. Le diagnostic peut être effectué avec plusieurs capteurs 4 et 5. Le dispositif d'analyse de posture 14 permet de déterminer si le porteur 2 est debout, assis ou couché en comparant les accélérations mesurées par les accéléromètres 4. Si le signal le plus grand est mesuré par l'accéléromètre 4 en X ou l'accéléromètre 4 en Y, le porteur est couché mais il est assis ou debout si l'accélération en Z est prépondérante puisque la gravité est dirigée suivant cet axe. Le diagnostic de posture est établi si les rapports des accélérations sont supérieurs à certains coefficients. Si le porteur 2 est debout, la comparaison des mesures pour les magnétomètres 5 en X et en Y peut donner sa direction dans les points cardinaux. La chute peut être déterminée par une rotation rapide autour d'un axe vertical ou une accélération rapide en rotation par rapport au champ de pesanteur (mesuré avec un accéléromètre). D'autres critères seront facilement déduits pour d'autres postures.

Le dispositif d'analyse du niveau d'activité 15 est destiné à indiquer si l'activité du porteur 2 atteint un niveau au-delà duquel les résultats obtenus pour les mesures physiologiques sont jugés comme impossibles à obtenir correctement. Il peut consister en un filtre de dérivation appliqué aux signaux des capteurs 4 et 5 et fournit une sortie binaire. Si un signal dérivé est supérieur à un seuil, ce qui traduit une variation trop brutale de mouvement, le dispositif 15 fournit une sortie nulle, sinon la sortie est égale à l'unité. Une autre façon de procéder consistera à appliquer un critère glissant sur les signaux dérivés provenant des capteurs, selon la formule ci-dessous :

$$\text{CRI} = \text{Abs}\left[d(t)-d(t-k)\right]Signe\left[d(t).d(t-k)\right]$$

où CRI est le critère, Abs l'opérateur de valeur absolue, d le signal dérivé provenant d'un capteur, t le temps, k une constante prédéfinie et Signe l'opérateur signe ; le dispositif 15 aura une sortie nulle si le résultat du calcul est inférieur à un seuil négatif, ce qui correspond à une inversion rapide du sens du mouvement, et une sortie égale à l'unité sinon.

Quand le signal du dispositif 15 est nul, le module de validation 18, qui est un multiplicateur, sort un signal nul et inhibe donc les calculs de l'activité physiologique ; sinon, quand le dispositif 15 émet un signal égal à 1, le module de validation 18 n'a pas d'influence sur le signal le traversant et le laisse passer sans le modifier.

Le dispositif d'estimation 16 a pour but d'isoler une composante du signal de chaque capteur 4 ou 5 qui est représentative de l'activité du porteur. Il peut s'agir d'un filtre comme un filtre passe-bas, ou, de façon plus intéressante,

3

d'un filtre non-stationnaire permettant de ne pas filtrer le signal en présence de point de rebroussement du signal correspondant à une inversion rapide de son évolution.

Un filtre F utilisant une fonction sigmoïde peut être utilisé. Ce procédé repose sur la notion que le signal peut être filtré sans inconvénient quand il est stable, mais qu'il ne doit pas l'être dans des situations de grande instabilité où l'activité du porteur comporte aussi des mouvements de plus haute fréquence.

Une fonction sigmoïde tend vers 0 pour des valeurs d'entrée proches de 0 et tend vers 1 pour des valeurs d'entrée très grandes. Un exemple est $1/(1+e^{-x})$, mais des fonctions comme arcsin, arctg, etc. en sont d'autres.

D'après ce qui précède, un filtre du signal d'entrée noté s(t) peut être un filtre passe-bas pondéré par le critère CRI rencontré plus haut :

$$F[S(t)] = \text{sigmoïde (CRI)}.s(t)+$$

$$(1-\text{sigmoïde (CRI)})xF[S(t) \text{ passe-bas}].$$

où la sigmoïde est $1/(1+e^{-x})$

D'autres fonctions de filtrage que F peuvent aussi être appliquées, de même que des filtres permettant d'extraire une composante à basse fréquence du signal qui maintienne les discontinuités. Un autre exemple de filtrage préconisé est celui qui est proposé dans l'article "Non linear anisotropic filtering of MRI data" IEEE Transactions on Médical Imaging, vol.11, n°2, p.231-232 par G. Gerig.

Le soustracteur 17 a une borne positive qui reçoit le signal normalisé et une borne négative qui reçoit le signal fourni par le dispositif d'estimation 16. La différence correspond au signal représentatif de l'activité physiologique. Comme on l'a vu, le module de validation 18 est un multiplicateur qui laisse inchangé ce signal dans des circonstances jugées normales et l'annule autrement. Le dispositif de choix 19 permet de choisir les signaux qui sont les plus représentatifs du mouvement de la respiration en fonction de la posture du porteur 2 estimée par le dispositif d'analyse de posture 14. Si le porteur 2 est couché, les mouvements dus à la respiration seront estimés par les accéléromètres 4 sensibles dans la direction Y et Z, et par les magnétomètres 5 dans les directions X et Z ; sinon, le porteur 2 étant debout ou assis, on considérera les accéléromètres 4 dans les directions X et Z et les magnétomètres 5 dans directions Y et Z. On écartera par ce moyen les accéléromètres influencés par l'accélération de la gravité et qui fourniraient des mesures trop bruitées.

L'extracteur de fréquence cardiaque 20 est un filtre passe-bande dont les limites sont, par exemple, 0,5 Hertz et 3 Hertz. Le dispositif 21 de calcul de la fréquence du coeur utilise avantageusement les accéléromètres 4 et notamment celui qui est orienté dans la direction X. La période est calculée en détectant les maximums consécutifs et en estimant les durées qui les séparent. Ces maximums sont produits par l'impulsion principale du coeur ; ils ont une largeur d'environ 30 millisecondes et sont séparés en moyenne par une durée d'environ 0,8 seconde pour une personne au repos. La détection pourra être améliorée en appliquant des filtrages adaptés à la forme des maximums à détecter, par exemple un filtre de largeur équivalente à 250 millisecondes qui est à une valeur égale à 1 au centre sur une largeur équivalente de 30 millisecondes, et 0 en périphérie. La fréquence du coeur est égale à l'inverse de la durée séparant les maximums. Un calcul de moyenne glissante pourra être effectué en prenant en considération la moyenne de quelques fréquences mesurées auparavant.

Le dispositif de sortie 22 est généralement un émetteur dirigeant les résultats obtenus vers un dispositif de visualisation ou de diagnostic extérieur au détecteur 1.

Le dispositif d'extraction de la composante respiratoire 23 consiste aussi en un filtre passe-bande entre les fréquences par exemple 0,03 hertz et 1 hertz. Le dispositif de calcul de fréquence respiratoire 24 utile les résultats d'un ou plusieurs capteurs 4 et 5 et calcule la fréquence respiratoire en estimant la durée entre trois passages par zéro consécutifs d'un signal respiratoire ; la fréquence est l'inverse de cette durée. Ici encore un calcul de moyenne glissante pourra être effectué pour améliorer les résultats, ou une moyenne de calcul sur plusieurs capteurs 4 et 5. Enfin, le dispositif de sortie 25 est encore un émetteur des résultats obtenus vers un moyen extérieur de visualisation ou diagnostic, ou encore un moyen de synchronisation d'un autre appareil sur le cycle respiratoire.

Il n'est pas nécessaire de placer six capteurs de mouvement dans le détecteur 1 pour utiliser l'invention mais il est manifeste que la mesure des mouvements dans toutes les directions par deux séries de capteurs ayant des références différentes donne des résultats plus universels.

Ces magnétomètres pourront être des sondes différentielles (fluxgates) ou des magnéto-résistances géantes.

Dans un autre mode de réalisation, le détecteur comprend une pluralité de capteurs répartis par exemple en différents endroits du corps, chaque capteur étant relié à l'unité de traitement 6 de signaux par exemple, par connexion électrique, par radiofréquence. L'avantage de ce mode de réalisation est de pallier l'incapacité d'un capteur à donner une information physiologique, par exemple dans le cas om le patient est appuyé sur un capteur, qui ne peut plus alors mesurer la respiration. On utilise les autres capteurs situés ailleurs. Le nombre de capteurs utilisés, leur degré de redondance et leurs emplacements ne sont pas critiques.

**Revendications**

**1.** Détecteur portatif (1) comprenant au moins un capteur (4, 5) recueillant un signal de mouvement d'une personne porteuse (2) du détecteur, comprenant des moyens de traitement du signal (6) permettant de distinguer une composante du signal due à une activité extérieure de la personne et au moins une composante du signal due à une activité physiologique de la personne, **caractérisé en ce qu'**il comprend un module de décision d'invalidation (15) d'estimation de la composante due à l'activité physiologique d'après un critère dépendant de la composante d'activité extérieure.

**2.** Détecteur selon la revendication 1, **caractérisé en ce qu'**il comprend un filtre, recevant le signal de mouvement, pour estimer la composante due à l'activité extérieure, et un module soustracteur (17), recevant le signal du mouvement à une borne positive et le signal issu du filtre (16) à une borne négative pour estimer la composante due à l'activité physiologique.

**3.** Détecteur selon l'une quelconque des revendications 1 à 2, **caractérisé en ce qu'**il comprend trois capteurs (4 ou 5) recueillant trois signaux de mouvements perpendiculaires, dont un mouvement d'avance, un mouvement de côté et un mouvement d'élévation de la personne.

**4.** Détecteur selon l'une quelconque des revendications 1 à 3, **caractérisé en ce qu'**il comprend une pluralité de capteurs recueillant des signaux de mouvement respectifs dont des magnétomètres, un module d'estimation de posture (14) de la personne, et un moyen de sélection de capteurs pour choisir certains des signaux de mouvement, qui seront appliqués aux moyens de traitement, et écarter d'autres des signaux de mouvement, en fonction de la posture de la personne.

**5.** Détecteur selon la revendication 2, **caractérisé en ce que** le filtre est non-stationnaire.

**6.** Capteur de chute **caractérisé en ce qu'**il comporte un détecteur selon l'une quelconque des revendications 1 à 5.

**Claims**

**1.** Portable detector (1) comprising at least one sensor (4, 5) collecting a movement signal from a person (2) wearing the detector, comprising signal processing means (6) for distinguishing a component of the signal due to an external activity of the wearer and at least one component of the signal due to a physiological activity of the wearer, **characterized in that** it comprises an invalidation decision module (15) for estimating the component due to the physiological activity based on a criterion that depends on the external activity component.

**2.** Detector according to claim 1, **characterized in that** it comprises a filter receiving the movement signal, to estimate the component due to external activity, and a subtracting module (17), receiving the movement signal at a positive terminal and the signal output from the filter (16) at a negative terminal to estimate the component due to the physiological activity.

**3.** Detector according to either of claims 1 or 2, **characterized in that** it comprises three sensors (4 or 5) collecting three perpendicular movement signals of the person, including one forward movement, one sideways movement and one upward movement.

**4.** Detector according to any one of claims 1 to 3, **characterized in that** it comprises several sensors collecting corresponding movement signals including magnetometers, a wearer posture estimating module (14), and a sensor selection means to select some movement signals, that will be applied to processing means, and discard other movement signals, as a function of the wearer's posture.

**5.** Detector according to claim 2, **characterized in that** the filter is non-stationary.

**6.** Fall sensor **characterized in that** it comprises a detector according to any one of claims 1 to 5.

**Patentansprüche**

1. Tragbarer Detektor (1) mit mindestens einem Sensor (4, 5) zur Aufnahme eines Bewegungssignals einer Detektorträgerperson (2), der Signalverarbeitungseinrichtungen (6) umfasst, die ermöglichen, zu unterscheiden zwischen einer auf einer äußerlichen Aktivität der Person beruhenden Komponente des Signals und einer auf einer physiologischen Aktivität der Person beruhenden Komponente des Signals,
**dadurch gekennzeichnet, dass** er einen Annullierungsentscheidungsmodul (15) zur Bewertung der auf der physiologischen Aktivität beruhenden Komponente nach einem von der Komponente der äußerlichen Aktivität abhängigen Kriterium umfasst.

2. Detektor nach Anspruch 1, **dadurch gekennzeichnet, dass** er ein Filter umfasst, welches das Bewegungssignal erhält, um die auf der äußerlichen Aktivität beruhende Komponente zu bewerten, sowie einen Subtraktionsmodul (17), der das Bewegungssignal an einem positiven Anschluss und das von dem Filter (16) stammende Signal an einem negativen Anschluss erhält, um die auf der physiologischen Aktivität beruhende Komponente zu bewerten.

3. Detektor nach einem der Ansprüche 1 und 2, **dadurch gekennzeichnet, dass** er drei Sensoren (4 oder 5) umfasst, die Signale von drei zueinander senkrechten Bewegungen empfangen, darunter eine Vorwärtsbewegung, eine Seitenbewegung und eine Aufwärtsbewegung der Person.

4. Detektor nach einem der Ansprüche 1 bis 3, **dadurch gekennzeichnet, dass** er eine Vielzahl von jeweils Bewegungssignale empfangenden Sensoren, darunter Magnetometer, einen die Körperhaltung der Person bewertenden Bewertungsmodul (14) und eine Sensorenselektionseinrichtung umfasst, um - in Abhängigkeit von der Körperhaltung der Person - bestimmte Bewegungssignale auszuwählen, die dann in die Verarbeitungseinrichtungen eingespeist werden, und andere Bewegungssignale auszuschließen.

5. Detektor nach Anspruch 2, **dadurch gekennzeichnet, dass** das Filter nichtstationär ist.

6. Sturzsensor, **dadurch gekennzeichnet, dass** er einen Detektor nach einem der Ansprüche 1 bis 5 umfasst.

FIG. 1

FIG. 2

FIG. 3